(19) Europäisches Patentamt
European Patent Office
Office européen des brevets

(11) Veröffentlichungsnummer: **0 046 138**
**B1**

(12) **EUROPÄISCHE PATENTSCHRIFT**

(45) Veröffentlichungstag der Patentschrift: **04.12.85**

(51) Int. Cl.[4]: **C 07 D 333/36,** C 07 D 409/12 // C07D307/32

(21) Anmeldenummer: **81810314.5**

(22) Anmeldetag: **03.08.81**

(54) **Verfahren zur Herstellung von 3-(N-Arylamino)-tetrahydrothiophen-2-on-derivaten.**

(30) Priorität: **08.08.80 US 176821**

(43) Veröffentlichungstag der Anmeldung: **17.02.82 Patentblatt 82/7**

(45) Bekanntmachung des Hinweises auf die Patenterteilung: **04.12.85 Patentblatt 85/49**

(84) Benannte Vertragsstaaten: **AT BE CH FR GB IT LI LU NL SE**

(56) Entgegenhaltungen:
**CHEMICAL ABSTRACTS, Band 51, Nr. 3, 10. Februar 1957, Spalte 1838g Columbus, Ohio, U.S.A. LENNART SCHOTTE: "Gamma-Butyrolactonereactions involving ring fission"**

(73) Patentinhaber: **CIBA-GEIGY AG, Postfach, CH-4002 Basel (CH)**

(72) Erfinder: **Kunz, Walter, Dr., Im Goldbrunnen 55, CH-4104 Oberwil (CH)**

**EP 0 046 138 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Die vorliegende Erfindung betrifft ein neues Verfahren zur Herstellung von 3-(N-Arylamino)-tetrahydrothiophen-2-on-derivaten durch Umsetzung von 3-(N-Arylamino)-tetrahydrofuran-2-on-derivaten mit Salzen von Thiocarbonsäuren. 3-(N-Arylamino)-tetrahydrothiophen-2-on-derivate sind aus DE-OS 2 845 454 (1979) und US-PS 4 165 322 (1979) als Fungizide bekannt.

In diesen Referenzen wird vorgeschlagen, daß man 3-(Arylamino)-tetrahydrothiophen-2-on-derivate durch Reaktion von N-Arylaminen mit 3-Halogentetrahydrothiophen-2-on-derivaten herstellen kann. Letztere lassen sich, falls gewünscht, mit Acylhalogeniden N-acylieren.

Diese bekannte Verfahrensweise ist aufwendig und für eine technische Herstellung wenig geeignet, insbesondere durch die Verwendung von 3-Halogen-tetrahydrothiophen-2-on-derivaten als Ausgangsprodukte, da ihre Herstellung über die Tetrahydrofuran-2-on-derivate nur mit großem präparativen Aufwand, unter Ausbeuteverlust oder mit starker Umweltbelastung zu verwirklichen ist.

Wie beispielsweise aus (CA. 84, 17 124 a [Japan Kokai 7 570 354 (31.10.1973)]) bekannt ist, führt die Halogenierung von Tetrahydrothiophen-2-on überwiegend zu 3,3-Dihalogen-tetrahydrothiophen-2-on und nur mit 13% Ausbeute zu dem gewünschten 3-Monohalogen-tetrahydrothiophen-2-on.

Die Tetrahydrothiophen-2-on-derivate selbst können zwar nach einer ganzen Reihe von bekannten Methoden hergestellt werden, aber alle diese Methoden sind mit bestimmten Nachteilen verbunden.

Es ist z. B. bekannt [Houben-Weyl 6/2, 851 bzw. DE-PS 809 557 (1949) BASF], daß Tetrahydrothiophen-2-on durch Reaktion von Tetrahydrofuran-2-on und Schwefelkohlenstoff hergestellt werden kann:

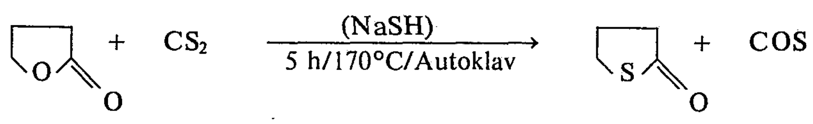


Diese Reaktion hat nicht nur den Nachteil, daß das stark toxische $CS_2$ als Ausgangsstoff verwendet wird und ebenfalls toxisches COS entsteht; sie ist auch nur unter hohen Drucken und unter hohem Energieaufwand durchführbar.

Eine weitere bekannte Verfahrensweise [Toland und Campbell, J. Org. Chem. 28, 3124 (1963)] zur Herstellung von Tetrahydrothiophen-2-onen geht von Dicarbonsäureanhydriden z. B. Bernsteinsäureanhydrid und dem toxischen Schwefelwasserstoff aus:

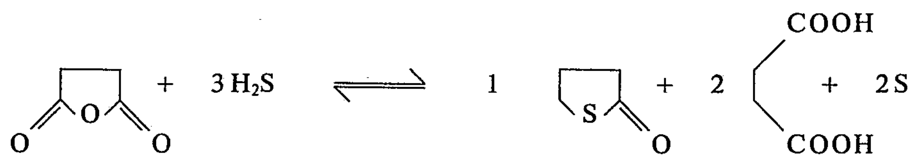

Hierbei entsteht auf 3 Moleküle des Ausgangsproduktes nur ein Molekül des Endproduktes, was einem Ausbeuteverlust von 2/3 entspricht.

Nach Reppe [Liebigs Annalen der Chemie 596, 173 (1955)] kann Tetrahydrothiophen-2-on aus Tetrahydrofuran-2-on hergestellt werden:

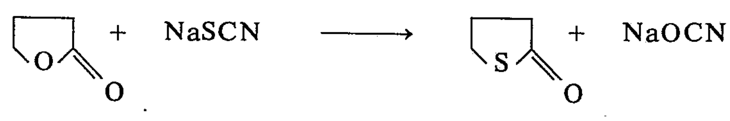

Bei dieser Reaktion wird toxisches Thiocyanid eingesetzt, und es entsteht, leichtlösliches umweltgefährdendes Cyanat.

Weiter ist bekannt [Korte u. Lohmer, Chem. Ber. 90, 1290 (1957)], daß Tetrahydrothiophen-2-on auch in einem komplizierten, mehrstufigen Verfahren hergestellt werden kann, indem zuerst Thioessigsäure an Vinylessigsäure addiert wird, dann das Acetylprodukt zu $\gamma$-Mercaptobuttersäure alkalisch hydrolysiert und schließlich die Säure bei hoher Temperatur zu Tetrahydrothiophen-2-on cyclisiert wird.

Die zum Verfahren vorliegender Erfindung nächstvergleichbare Reaktion von Tetrahydrofuran-2-on mit Alkalisalzen der Thioessigsäure [Schotte, Arkiv Kemi 8, 457–61; (C. A. 51, 1838 h)] führt zu einer Öffnung des Tetrahydrofuranringes:

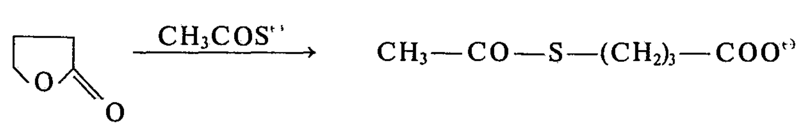

Es bildet sich das $\gamma$-Acetylmercapto-buttersäuresalz, das durch Neutralisation in die freie Säure überführt werden kann. Soweit aus dem beschreibenden und präparativen Teil ersichtlich,führt Schotte die Reaktionen in wäßriger Lösung durch.

Es wurde nun völlig überraschend gefunden, daß man beim Einsatz von 3-(N-arylamino)-tetrahydrofuran-2-on-Derivaten an Stelle des Tetrahydrofuran-2-ons und Reaktion mit dem Salz einer Thiocarbonsäure in organischen Lösungsmitteln nicht etwa das nach Schotte zu erwartende offenkettige $\gamma$-Acetylmercaptoessigsäurederivat, sondern auf besonders einfache Weise und in hoher Ausbeute 3-(N-Arylamino)-tetrahydrothiophen-2-on-Derivate erhält.

Das Verfahren gemäß vorliegender Erfindung betrifft die Herstellung von 3-(N-Arylamino)-tetrahydrothiophen-2-on-derivaten der allgemeinen Formel A

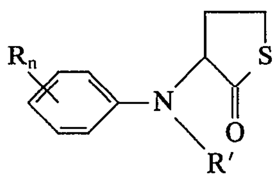

(A)

worin der heterocyclische Fünfring unsubstituiert oder ein- oder mehrfach durch $C_1$—$C_4$-Alkyl substituiert ist, n für eine ganze Zahl von 0 bis 5 steht, R eine durch n bestimmte Anzahl von Substituenten $C_1$—$C_{12}$-Alkyl, $C_1$—$C_{12}$-Alkoxy, Halogen, Nitro oder $-CH_2-X-R''$ bedeutet, oder worin zwei Substituenten R nachbarständig zueinander zusammen mit der Phenylgruppe einen Naphthylring bilden, wobei X Sauerstoff oder Schwefel bedeutet, R″ für $C_1$—$C_{12}$-Alkyl, Phenyl oder Benzyl steht, von denen jeder der drei unsubstituiert oder ein- oder mehrfach durch Niederalkyl, Niederalkoxy, Halogen oder Nitro substituiert ist, und R′ für Wasserstoff oder die Gruppe $-CO-R'''$ steht, wobei R‴ einen aliphatischen, heterocyclisch-aliphatischen oder heterocyclischen Rest bedeutet, wobei der Heterocyclus jeweils ein- oder mehrere N-, O- und/oder S-Atome enthält, oder wobei R‴ einen aromatischen oder araliphatischen Rest bedeutet, von denen jeder im aromatischen Teil halogeniert sein kann, und wobei eine aliphatische Kette in irgendeinem der Substituenten R‴ ein- oder mehrfach durch Sauerstoff, Schwefel, oder Stickstoff unterbrochen sein kann; und ist gekennzeichnet durch die Reaktion einer Verbindung der Formel B

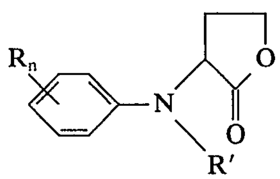

(B)

worin die möglichen Substituenten die unter Formel A angegebenen Bedeutungen haben, in einem organischen Lösungsmittel bei Temperaturen von ca. +15 bis +200°C mit einem Salz einer Thiocarbonsäure der Formel III

$$R-COS-X \qquad (III)$$

worin R einen substituierten oder unsubstituierten aliphatischen oder aromatischen Rest und X ein Kation aus der ersten bis vierten Hauptgruppe oder der ersten bis achten Nebengruppe des Periodensystems bedeuten.

Unter Niederalkyl oder Niederalkylanteil eines anderen Substituenten soll ein geradkettiger oder verzweigter Alkylrest mit maximal 6 Kohlenstoffatomen verstanden werden, beispielsweise Methyl, Ethyl, Propyl oder Butyl sowie ihre Isomeren, wie z. B. Isopropyl, Isobutyl, tert.-Butyl usw. Halogen steht hier und im folgenden für Fluor, Chlor, Brom oder Jod, vorzugsweise für Fluor, Chlor oder Brom.

Typische Beispiele für araliphatische Substituenten sind Benzyl, Phenylethyl, Phenylethenyl und 2,2-Diphenylethenyl. Heterocyclisch-aliphatische Reste sind z. B. Triazolylmethyl, Imidazolylethyl und 2-Methylpyridyl.

Heterocyclische Substituenten bzw. heterocyclische Anteile von Substituenten leiten sich insbesondere von Fünf- oder Sechsring-Systemen ab und können im Ring eine oder mehrere Doppelbindungen tragen oder vollständig hydriert sein. Als vorteilhafte Reste seien genannt: Isoxazol, Furan, Tetrahydrofuran, $\alpha$-Pyran, $\gamma$-Pyran, Dihydropyran, Thiophen, Tetrahydrothiophen, Pyrrol, Pyrrolidin, Pyrazol, Imidazol, Triazol, Piperidin, Piperazin, Pyridin, Pyrimidin, Pyridazin, Pyrazin, Triazin, Morpholin, Thiazin, Thiomorpholin und benzokondensierte Systeme wie Chinolin, Isochinolin, 1,2-Dihydrochinolin, Acridin, Chromen, Chroman, Indol, Benzofuran, Benzothiophen und Benzimidazol.

Unter der Bezeichnung Salz soll vor allem ein Metall-, Ammonium-, Hydrazinium- oder sonstiges Salz einer quaternierten organischen N-haltigen Base zu verstehen sein.

Das erfindungsgemäße Verfahren ist insbesondere zur Herstellung von 3-(N-Anilino)-tetrahydrothiophen-2-on-Derivaten der Formel I geeignet

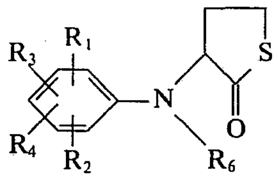

(I)

worin

$R_1$ Wasserstoff, $C_1$—$C_4$-Alkyl, $C_1$—$C_4$-Alkoxy oder Halogen,
$R_2$ Wasserstoff, $C_1$—$C_4$-Alkyl, $C_1$—$C_4$-Alkoxy, Halogen oder Nitro,
$R_3$ Wasserstoff, $C_1$—$C_4$-Alkyl, $C_1$—$C_4$-Alkoxy, Halogen oder die Gruppe $CH_2$—X—$R_8$ bedeuten, wobei $R_8$ für $C_1$—$C_4$-Alkyl, Phenyl oder Benzyl und X für Sauerstoff, oder Schwefel stehen,
$R_4$ Wasserstoff oder $C_1$—$C_4$-Alkyl,
$R_6$ Wasserstoff oder die Gruppe —CO—$R_7$ bedeutet, wobei $R_7$ für einen gesättigten oder ungesättigten aliphatischen Kohlenwasserstoffrest mit maximal 4 Kohlenstoffatomen oder eine Alkylthioalkyl- oder Alkoxyalkylgruppe mit 2 bis 5 Kohlenstoffatomen, eine durch Halogen substituierte oder unsubstituierte 2-Furyl- oder 2-Tetrahydrofurylgruppe, eine 1,2,4-Triazolylmethyl- oder Cyclopropylgruppe steht,

und ist gekennzeichnet durch die Reaktion eines 3-(N-anilino)-tetrahydrofuran-2-on-derivats der Formel II

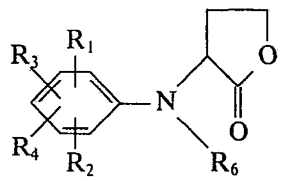

(II)

worin $R_1$, $R_2$, $R_3$, $R_4$ und $R_6$ die unter Formel I angegebenen Bedeutungen haben, in einem organischen Lösungsmittel mit einem Thiocarbonsäuresalz der Formel III

R—COSX (III)

worin R für eine substituierte oder unsubstituierte Alkyl- oder Arylgruppe steht und X ein Metall- oder ein Ammoniumion bedeutet.

Ausgangsverbindungen der Formel II sind zum überwiegenden Teil aus folgenden Publikationen bekannt:

DE-OS 2 804 299
DE-OS 2 845 454
US-PS 4 165 322

Ihre Herstellung wird dort beschrieben. Sonstige Zwischenprodukte werden analog hergestellt bzw. sind in der im Jahre 1980 publizierten EP-Anmeldung Nr. 18 510 beschrieben.

Salze von aliphatischen und aromatischen Thiocarbonsäuren sind allgemein bekannt oder werden nach allgemein bekannten Methoden hergestellt.

Es zeigt sich überraschend, daß bei der Umsetzung eines 3-(N-Arylamino)-tetrahydrofuran-2-ons mit dem Salz einer Thiocarbonsäure die Struktur des Tetrahydrofuranonringes erhalten bleibt und lediglich ein formaler Sauerstoff-Schwefel-Austausch stattfindet. Dieser Reaktionsverlauf widerspricht völlig den Erwartungen und führt in einer einfachen Umsetzung zu den gewünschten Thioverbindungen der Formel A oder I.

3-(N-Arylamino)-tetrahydrothiophen-2-on-Derivate lassen sich somit auf eine sehr einfache, wirtschaftliche und umweltfreundliche Weise in guten Ausbeuten und unter Verwendung von leicht zugänglichen, billigen Ausgangsprodukten herstellen.

Es ist vorteilhaft, die Reaktion von 3-(N-Arylamino)-tetrahydrofuran-2-on-Derivaten der Formeln B oder II mit Salzen von Thiocarbonsäuren in Gegenwart eines organischen Lösungsmittels vorzunehmen. Geeignete organische Lösungsmittel sind solche, in denen die Salze der Thiocarbonsäuren ausreichend löslich sind und sich gegenüber den Ausgangsstoffen inert verhalten. Es eignen sich besonders aprotische Lösungsmittel und unter diesen, solche mit polarem Charakter. Beispiele vorteilhafter Lösungsmittel sind:

Amide, bevorzugt N-alkylierte Amide wie N,N-Dimethylformamid, N,N-Dimethylacetamid, N,N-Diethylacetamid, N,N-Dimethylmethoxyacetamid und Hexamethylphosphortriamid; Sulfoxide wie Dimethylsulfoxid und Diethylsulfoxid; Sulfone wie Tetrahydrothiophen-1,1-dioxid; Ethylenglykol- und

Diethylenglykoldiether und -monoether mit je 1–4 C-Atomen in den Alkylteilen, wie Ethylenglykoldimethyl-, -diethyl- und -di-n-butylether, Diethylenglykoldiethyl- und -di-n-butylether, Ethylenglykolmonomethylether und Diethylenglykolmonomethylether; etherartige Verbindungen wie Dioxan, Tetrahydrofuran und Tetrahydropyran und Gemische solcher Lösungsmittel untereinander.

Nimmt man längere Reaktionszeiten in Kauf, so lassen sich auch weniger polare und unpolare organische Lösungsmittel einsetzen; hierbei erweist sich der Zusatz eines üblichen Phasentransferkatalysators als vorteilhaft.

Beispiele derartiger Katalysatoren sind: Tetraalkylammoniumhalogenide, -hydrogensulfate oder -hydroxide wie Tetrabutylammoniumchlorid, -bromid, -jodid; Triethylbenzylammoniumchlorid, -bromid; Tetrapropylammoniumchlorid, -bromid, -jodid; usw. Als Phasentransfer-Katalysatoren kommen auch Phosphonium-Salze in Betracht.

Die Umsetzung von Verbindungen der Formeln B oder II mit Salzen von Thiocarbonsäuren nimmt je nach Reaktionstemperatur eine Reaktionszeit von bis zu fünfzig Stunden in Anspruch. In den meisten Fällen genügen Reaktionszeiten von einer bis zwanzig Stunden.

Die Reaktionstemperaturen können bevorzugt zwischen +50 und +200°C liegen, besonders bevorzugt ist ein Temperaturbereich von +100 bis +150°C.

Als Thiocarbonsäuresalze der Formel III

$$R—COSX \qquad (III)$$

kommen Salze der ersten bis vierten Hauptgruppenelemente sowie der ersten bis achten Nebengruppenelemente in Frage, insbesondere die Alkali- und Erdalkalisalze, bevorzugt die Alkalisalze. Sehr gut eignen sich auch substituierte und unsubstituierte Ammoniumsalze, z. B. Thiocarbonsäuresalze mit den Kationen: $NH_4^{\oplus}$, $NH_3(Alkyl)^{\oplus}$, $NH_2(Alkyl)_2^{\oplus}$, $NH(Alkyl)_3^{\oplus}$, $N(Alkyl)_4^{\oplus}$ wobei der Alkylanteil 1 bis 12 Kohlenstoffatome enthält. Geeignete salzbildende Thiocarbonsäuren sind beispielsweise substituierte und unsubstituierte aliphatische und aromatische Thiocarbonsäuren, wie z. B. Thiobenzoesäure, Phenylthioessigsäure, Diphenylthioessigsäure, Thioessigsäure, Thiopropionsäure, Thiobuttersäure, Thiovaleriansäure, Thionaphthoesäure usw. Bevorzugt sind aliphatische Thiocarbonsäuren, insbesondere solche mit einem Alkylanteil bis zu 6 Kohlenstoffatomen.

Beispiele derartiger Salze sind:

$CH_3COS^{\ominus}K^{\oplus}$ $CH_3COS^{\ominus}Na^{\oplus}$ $C_2H_5COS^{\ominus}K^{\oplus}$ $C_6H_5COS^{\ominus}Na^{\oplus}$ $CH_3COS^{\ominus}NH_4^{\oplus}$

$C_2H_5COS^{\ominus}NH_4^{\oplus}$ $CH_3COS^{\ominus}N(n\text{-}C_4H_9)_4^{\oplus}$

Unter Umständen kann es sich als Vorteil erweisen, wenn das Herstellungsverfahren unter Schutzgasatmosphäre, z. B. unter Stickstoff und in absolutierten Lösungsmitteln durchgeführt wird.

Herstellungsbeispiele:

Beispiel 1: Herstellung von

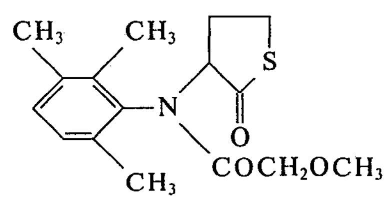

(5)

3-[N-(Methoxyacetyl)-N-(2,3,6-trimethylphenyl)]-amino -tetrahydrothiophen-2-on

14,5 g (0,05 Mol) 2-[N-(Methoxyacetyl)-N-(2,3,6-trimethylphenyl)]-amino -tetrahydrofuran-2-on und 7 g (0,05 Mol) Kaliumthioacetat werden unter Stickstoffatmosphäre in 50 ml Dimethylformamid gelöst und 4 Stunden bei 150°C gerührt. Nach Abziehen des Lösungsmittels im Vakuum wird das Reaktionsgemisch auf Eiswasser gegossen und mit Dichlormethan extrahiert. Die vereinigten Extrakte werden über Natriumsulfat getrocknet, filtriert, im Vakuum eingeengt und mit Diethylether/Petrolether versetzt. Aus der Lösung kristallisieren 10,5 g (68,4% der Theorie) farbloses 3-[N-(Methoxyacetyl)-N-(2,3,6-trimethylphenyl)]-amino -tetrahydrothiophen-2-on. Fp. 86–90°C.

Beispiel 2: Herstellung von

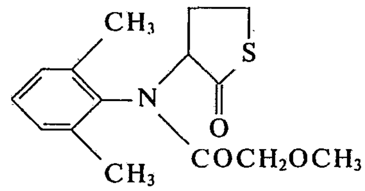

(1)

3-[N-(Methoxyacetyl)-N-(2,6-dimethylphenyl)]-amino -tetrahydrothiophen-2-on

110,8 g (0,4 Mol) 3-[N-(Methoxyacetyl)-N-(2,6-dimethylphenyl)]-amino -tetrahydrofuran-2-on und 39,4 g (0,4 Mol) Natriumthioacetat werden unter Stickstoffatmosphäre in 500 ml Dimethylformamid gelöst und 18 Stunden bei 100 bis 110°C gerührt. Nach Entfernen des Lösungsmittels im Vakuum wird das Reaktionsgemisch auf Eiswasser gegossen und mit Dichlormethan extrahiert. Die vereinigten Extrakte werden über Natriumsulfat getrocknet, filtriert, im Vakuum eingeengt und mit Diethylether versetzt. Aus der Lösung kristallisierten 58,4 g (49,8% der Theorie) farbloses 3-[N-(Methoxyacetyl)-N-(2,6-dimethylphenyl)]-amino -tetrahydrothiophen-2-on. Fp. 96—98°C.

Beispiel 3: Herstellung von

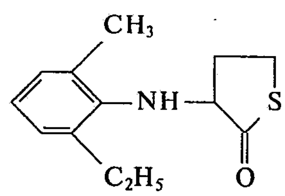

(18)

3-(N-2-Methyl-6-ethylphenyl)-amino-tetrahydrothiophen-2-on

a) 11 g (0,05 Mol) N-(2-Methyl-6-ethylphenyl)-amino-tetrahydrofuran-2-on und 4,7 g (0,05 Mol) Ammoniumthioacetat werden 80 ml Dimethylsulfoxid bei 90 bis 100°C gerührt. Nach dem Abkühlen wird die Lösung auf Eiswasser gegossen und mit Dichlormethan extrahiert. Die vereinigten Extrakte werden wiederholt mit Wasser gewaschen, über Natriumsulfat getrocknet, filtriert und eingeengt. Eine Hochvakuumdestillation des Rückstandes ergibt 5,7 g (48,5% der Theorie) farbloses N-(2-Methyl-6-ethylphenyl)-amino-tetrahydrothiophen-2-on. Kp 167—173°C bei 0,5 Torr.

b) 11 g (0,05 Mol) N-(2-Methyl-6-ethylphenyl)-amino-tetrahydrofuran-2-on und 6,6 g (0,05 Mol) Kaliumthioacetat werden in 200 ml Diethylenglykoldimethylether gelöst und 20 Stunden bei 150°C gerührt. Nach dem Abkühlen wird das Gemisch auf Eiswasser gegossen und mit Dichlormethan extrahiert. Die vereinigten Extrakte werden wiederholt mit Wasser gewaschen, über Natriumsulfat getrocknet, filtriert und eingeengt. Der Rückstand ergibt nach einer Hochvakuumdestillation 5,2 g (44,2% der Theorie) N-(2-Methyl-6-ethylphenyl)-amino-tetrahydrothiophen-2-on. Kp. 160—165°C bei 0,6 mbar.

Beispiel 4: Herstellung eines Zwischenproduktes

a) 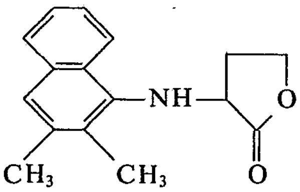

3-[N-(2,3-Dimethyl-$\alpha$-naphthyl)]-amino-tetrahydrofuran-2-on

114,3 g 2,3-Dimethyl-$\alpha$-naphthylamin, 143,6 g 3-Brombutyrolacton und 78,4 g $Na_2CO_3$ werden in 400 ml Dimethylformamid (= DMF) suspendiert. Die tiefgefärbte Suspension wird 15 Std. bei 75°C gerührt. Nach dem Abkühlen werden 300 ml Ethylacetat zugefügt und das Reaktionsgemisch abgesaugt. Das Filtrat wird eingeengt, und der verbleibende Rückstand wird mit Diethylether gewaschen und dann mit wenigen Millilitern einer 1 : 1-Mischung von Isopropanol/Aceton behandelt, wobei Kristallisation eintritt. Das so erhaltene kristalline Zwischenprodukt schmilzt bei 125—127°C.

b) Herstellung von

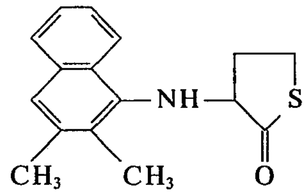

3-[N-(2,3-Dimethyl-α-naphthyl)]-amino-tetrahydrothiophen-2-on

12,2 g des gemäß a) erhaltenen Zwischenproduktes und 7,2 g Kalium-Thioacetat ($CH_3-CO-SK$) werden in 60 ml DMF suspendiert. Das Reaktionsgemisch wird 5 Std. bei 105—110 °C gerührt und am Hochvakuum eingeengt. Der Rückstand wird in Eiswasser gegossen und mit Methylenchlorid mehrfach extrahiert. Die vereinigten Extrakte werden mit Wasser gewaschen, über Na-Sulfat getrocknet und filtriert. Das Filtrat wird eingeengt. Der ölige Rückstand wird über eine Silicagel-Säule gereinigt, bei der Chloroform/Diethylether (1 : 1) als Laufmittel dient. Aus der erhaltenen klaren Lösung kristallisiert beim Eindampfen das Endprodukt aus, Smp. 81—84 °C.

Beispiel 5: Herstellung von

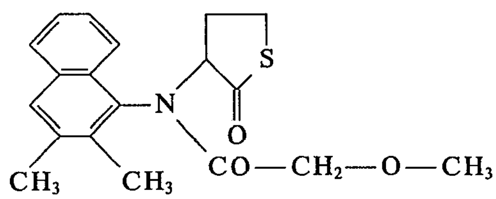

3-[N-(2,3-Dimethyl-α-naphthyl)-N-(methoxyacetyl)-amino-tetrahydrothiophen-2-on

9,7 g 3-[N-(2,3-Dimethyl-α-naphthyl)-N-(methoxyacetyl]-amino-tetrahydrofuran-2-on, das man durch Acylierung des gemäß Beispiel 4a erhaltenen Zwischenproduktes mit Methoxyacetylchlorid erhält, und 4,1 g Kalium-Thioacetat werden in 40 ml DMF suspendiert. Das Reaktionsgemisch wird $5^1/_2$ Std. bei 105—110 °C gerührt und dann im Vakuum eingeengt. Der erhaltene Rückstand wird nach der Methode des Beispiels 4b aufgearbeitet. Das kristalline Endprodukt schmilzt bei 140—144 °C.

Beispiel 6: Herstellung von

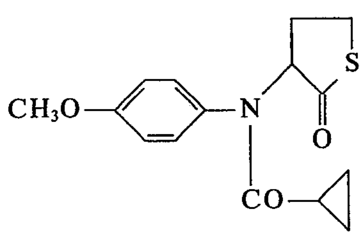

3-[N-(Cyclopropylcarbonyl)-N-(4-methoxyphenyl)]-amino-tetrahydrothiophen-2-on

7,2 g 3-[N-(Cyclopropylcarbonyl)-N-(4-methoxyphenyl)]-amino-tetrahydrofuran-2-on, das man konventionell durch Reaktion von 4-Methoxyanilin und 3-Brombutyrolacton sowie nachfolgende Acylierung des erhaltenen Zwischenproduktes mit Cyclopropancarbonsäurechlorid gewinnt, werden mit 3,7 g Kalium-Thioacetat in 30 ml DMF suspendiert. Das Reaktionsgemisch wird 5 Std. bei 105—110 °C gerührt, im Vakuum eingeengt und nach der Methode des Beispiels 4b aufgearbeitet. Das kristalline Endprodukt schmilzt bei 109—112 °C.

Beispiel 7: Herstellung von

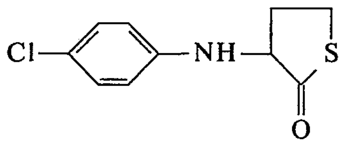

3-N-(4-Chlorphenyl)-amino-tetrahydrothiophen-2-on

15,0 g 3-N-(4-Chlorphenyl)-amino-tetrahydrofuran-2-on und 10,1 g Kalium-Thioacetat werden mit 70 ml DMF verrührt. Das Reaktionsgemisch wird unter Rühren 11 Std. auf 110°C erhitzt. Die Reaktionslösung wird im Vakuum eingeengt und nach der Methode des Beispiels 4b aufgearbeitet. Das kristalline Endprodukt schmilzt bei 86–90°C.

Entsprechend lassen sich z. B. folgende Verbindungen der Formel I herstellen:

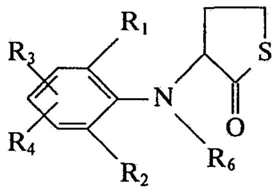

Wo in den Tabellen eine Seitenkette $R_6$ = —CO—Haloaliphat angegeben ist, wird die fragliche Verbindung vorzugsweise zuerst als nicht-acyliertes Zwischenprodukt mit $R_6$ = Wasserstoff hergestellt und dann auf übliche Art mit einer Carbonsäure der Formel

HO—CO—Haloaliphat

oder ihrem Säurehalogenid, Säureanhydrid oder Ester acyliert. In einigen Fällen läßt sich allerdings die erfindungsgemäße Umwandlung des Tetrahydrofuranon-Teils in den Tetrahydrothiophenon-Teil auch in Gegenwart einer haloaliphatischen Seitenkette $R_6$ vornehmen.

| Verb. Nr. | $R_1$ | $R_2$ | $R_3$ | $R_4$ | $R_6$ | Physik. Konst. Fp./Kp. [°C] |
|---|---|---|---|---|---|---|
| 1 | $CH_3$ | $CH_3$ | H | H | $COCH_2OCH_3$ | Fp. 96–98 |
| 2 | $CH_3$ | $CH_3$ | 3-$CH_3$ | 5-$CH_3$ | $COCH_2OCH_3$ | Fp. 123–125 |
| 3 | $CH_3$ | $CH_3$ | H | H | H | Kp. 140–145 (0,4 Torr) |
| 4 | $CH_3$ | $CH_3$ | 3-$CH_3$ | 5-$CH_3$ | CO— 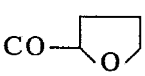 | Fp. 205–206 |
| 5 | $CH_3$ | $CH_3$ | 3-$CH_3$ | H | $COCH_2OCH_3$ | Fp. 86–90 |
| 6 | $CH_3$ | $CH_3$ | H | H | CO— 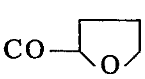 | Fp. 125–127 |
| 7 | $CH_3$ | $CH_3$ | 3-$CH_3$ | H | H | Kp. 173–183 (0,7 Torr) |
| 8 | $CH_3$ | $CH_3$ | 3-$CH_3$ | H | CO— 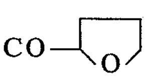 | Harz |
| 9 | $CH_3$ | $CH_3$ | 3-$CH_2O$—s.$C_4H_9$ | H | $COCH_2OCH_3$ | Harz |
| 10 | $CH_3$ | $CH_3$ | 3-$CH_2O$—s.$C_4H_9$ | H | H | Harz |
| 11 | $CH_3$ | $CH_3$ | 3-$CH_2O$—Benzyl | H | H | Harz |

Fortsetzung

| Verb. Nr. | $R_1$ | $R_2$ | $R_3$ | $R_4$ | $R_6$ | Physik. Konst. Fp./Kp. [°C] |
|---|---|---|---|---|---|---|
| 12 | $CH_3$ | $CH_3$ | 3-$CH_2O$—Benzyl | H | H | Harz |
| 13 | $C_2H_5$ | $C_2H_5$ | H | H | $COCH_2OCH_3$ | Fp. 51–54 |
| 14 | $CH_3$ | $CH_3$ | H | H | CO—Cyclopropyl | Fp. 115–117 |
| 15 | H | H | H | H | CO—Cyclopropyl | Fp. 81–83 |
| 16 | H | H | H | H | H | Kp. 145–160 (0,5 Torr) |
| 17 | $C_2H_5$ | $C_2H_5$ | H | H | CO—Cyclopropyl | Fp. 130–133 |
| 18 | $CH_3$ | $C_2H_5$ | H | H | H | Kp. 167–173 (0,5 Torr) |
| 19 | $CH_3$ | $C_2H_5$ | H | H | $COCH_2OCH_3$ | Fp. 125–133 |
| 20 | $C_2H_5$ | $C_2H_5$ | H | H | H | Kp. 162–173 (0,7 Torr) |
| 21 | $CH_3$ | $CH_3$ | 3-$CH_2OCH_3$ | H | $COCH_2OC_2H_5$ | Harz |
| 22 | $CH_3$ | $CH_3$ | 3-$CH_2OCH_3$ | H | $COCH_2Cl$ | Harz |
| 23 | $CH_3$ | $CH_3$ | 3-$CH_2OCH_3$ | H | H | Kp. 187–194 (0,5 Torr) |
| 24 | $CH_3$ | $CH_3$ | 3-$CH_3$ | H | CO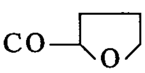 | Fp. 126–128 |
| 25 | $CH_3$ | $CH_3$ | 3-$CH_3$ | H | CO—Cyclopropyl | Fp. 130–132 |
| 26 | $CH_3$ | $CH_3$ | 3-$CH_3$ | H | $COCH_2Cl$ | Fp. 106–108 |
| 27 | $CH_3$ | $CH_3$ | H | H | CO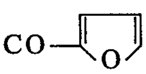 | Fp. 175–177 |
| 28 | $CH_3$ | $CH_3$ | H | H | $COCH_2Cl$ | Fp. 134–136 |
| 29 | $CH_3$ | $CH_3$ | H | H | $COCH_2OC_2H_5$ | Fp. 91–92 |
| 30 | $CH_3$ | $CH_3$ | H | H | CO—Cyclopropyl | Fp. 139–142 |
| 31 | $CH_3$ | $CH_3$ | 3-$CH_3$ | H | CO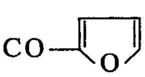 | Fp. 139–158 |
| 32 | $CH_3$ | $CH_3$ | 3-$CH_3$ | H | $COCH_2OC_2H_5$ | Fp. 90–95 |
| 33 | $CH_3$ | $CH_3$ | 3-$CH_2OCH_3$ | H | $COCH_2OCH_3$ | Harz |

| Verb. Nr. | $R_1$ | $R_2$ | $R_3$ | $R_4$ | $R_6$ |
|---|---|---|---|---|---|
| 34 | H | H | H | H | $COCH_2OCH_2CH{=}CH_2$ |
| 35 | $CH_3$ | $CH_3$ | H | H | $COCH_2OCH_2CH{=}CH_2$ |
| 36 | $CH_3$ | $CH_3$ | H | H | $COCH_2OCH_2C{\equiv}C{-}CH_3$ |
| 37 | H | H | H | H | $COCH_2SCH_3$ |
| 38 | $CH_3$ | $CH_3$ | H | H | $COCH_2SCH_3$ |

Die folgenden Verbindungen sollten sich auch in der acylierten Form nach dem erfindungsgemäßen Verfahren herstellen lassen, sonst aber von der Stufe des Zwischenprodukts ($R_6 = H$) ausgehend mit anschließender konventioneller Nachacylierung.

| Verb. Nr. | $R_1$ | $R_2$ | $R_3$ | $R_4$ | $R_6$ |
|---|---|---|---|---|---|
| 39 | $CH_3$ | $CH_3$ | H | H | $COCH_2OCH_2C{\equiv}CH$ |
| 40 | $CH_3$ | $CH_3$ | 3-$CH_3$ | 5-$CH_3$ | $COCH_2OCH_2C{\equiv}CH$ |
| 41 | $CH_3$ | $CH_3$ | 3-Cl | H | $COCH_2OCH_2C{\equiv}CH$ |
| 42 | $CH_3$ | $NO_2$ | H | H | $COCH_2OCH_2C{\equiv}CH$ |
| 43 | $CH_3$ | Cl | H | H | $COCH_2OCH_2C{\equiv}CH$ |
| 44 | $CH_3$ | H | 5-$CH_3$ | H | $COCH_2OCH_2C{\equiv}CH$ |

Die folgenden Verbindungen der Formel A

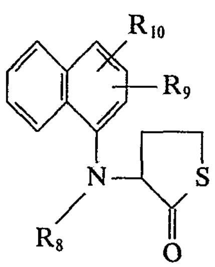

lassen sich analog herstellen.

| Verb. Nr. | $R_8$ | $R_9$ | $R_{10}$ | Smp. (°C) |
|---|---|---|---|---|
| 2.1 | $COCH_2OCH_3$ | 2-$CH_3$ | H | |
| 2.2 | $COCH_2OCH_3$ | 2-$CH_3$ | 3-$CH_3$ | 140–144 |
| 2.3 | H | 2-$CH_3$ | 3-$CH_3$ | 81–84 |
| 2.4 | $COCH_2OCH_3$ | 2-$C_2H_5$ | 3-$C_2H_5$ | |

Fortsetzung

| Verb. Nr. | $R_8$ | $R_9$ | $R_{10}$ | Smp. (°C) |
|---|---|---|---|---|
| 2.5 | $COCH_2OCH_3$ | 2-$C_2H_5$ | H | |
| 2.6 | —CO— 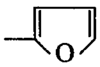 | 2-$CH_3$ | H | |
| 2.7 | —CO— 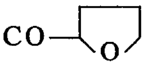 | 2-$CH_3$ | 3-$CH_3$ | |
| 2.8 | $COCH_2OCH_2—C{\equiv}C—CH_3$ | 2-$CH_3$ | H | |
| 2.9 | $COCH_2OCH_2CH{=}CH_2$ | 2–$CH_3$ | H | |
| 2.10 | $COCH_2OCH_2CH{=}CH_2$ | 2-$CH_3$ | 3-$CH_3$ | |

Die folgenden Verbindungen sollten sich auch in der acylierten Form nach dem erfindungsgemäßen Verfahren herstellen lassen, sonst aber ausgehend von der Stufe des Zwischenprodukts ($R_8$ = H) mit anschließender Nachacylierung.

| Verb. Nr. | $R_8$ | $R_9$ | $R_{10}$ |
|---|---|---|---|
| 2.11 | $COCH_2OCH_2—C{\equiv}CH$ | 2-$CH_3$ | H |
| 2.12 | $COCH_2OCH_2—C{\equiv}CH$ | 2-$CH_3$ | 2-$CH_3$ |
| 2.13 | $COCH_2OCH_2—C{\equiv}CH$ | 2-$NO_2$ | H |
| 2.14 | $COCH_2OCH_2—C{\equiv}CH$ | 2-$CH_3$ | 4-$NO_2$ |

Das neue Verfahren der vorliegenden Erfindung ist nicht auf die Herstellung von Verbindungen der Formel A oder der Formel I beschränkt. Der Fachmann wird die Lehre dieser Erfindung leicht auf die Herstellung weiterer substituierter Thiolactone aus substituierten Lactonen anwenden können.

## Patentansprüche

1. Verfahren zur Herstellung von 3-(N-Arylamino)-tetrahydrothiophen-2-on-derivaten der allgemeinen Formel A

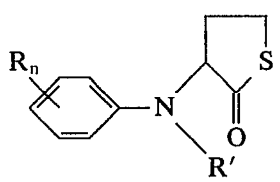

(A)

worin der heterocyclische Fünfring unsubstituiert oder ein- oder mehrfach durch $C_1$–$C_4$-Alkyl substituiert ist, n für eine ganze Zahl von 0 bis 5 steht, R eine durch n bestimmte Anzahl von Substituenten $C_1$–$C_{12}$-Alkyl, $C_1$–$C_{12}$-Alkoxy, Halogen, Nitro oder $-CH_2-X-R''$ bedeutet, oder worin zwei Substituenten R nachbarständig zueinander zusammen mit der Phenylgruppe einen Naphthylring bilden, wobei X Sauerstoff oder Schwefel bedeutet, R″ für $C_1$–$C_{12}$-Alkyl, Phenyl oder Benzyl steht, von denen jeder der drei unsubstituiert oder ein- oder mehrfach durch Niederalkyl, Niederalkoxy, Halogen oder Nitro substituiert ist, und R′ für Wasserstoff oder die Gruppe —CO—R‴ steht, wobei R‴ einen aliphatischen, heterocyclisch-aliphatischen oder heterocyclischen Rest bedeutet, wobei der Heterocyclus jeweils ein- oder mehrere N-, O- und/oder S-Atome enthält, oder wobei R‴ einen aromatischen oder araliphatischen

Rest bedeutet, von denen jeder im aromatischen Teil halogeniert sein kann und wobei eine aliphatische Kette in irgendeinem der Substituenten R''' ein- oder mehrfach durch Sauerstoff, Schwefel, oder Stickstoff unterbrochen sein kann; gekennzeichnet durch die Reaktion einer Verbindung der Formel B

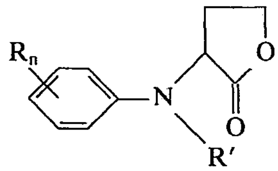

(B)

worin die möglichen Substituenten die unter Formel A angegebenen Bedeutungen haben, in einem organischen Lösungsmittel bei Temperaturen von ca. +15 bis +200°C mit einem Salz einer Thiocarbonsäure der Formel III

$$R—COS—X \quad (III)$$

worin R einen substituierten oder unsubstituierten aliphatischen oder aromatischen Rest und X ein Kation aus der ersten bis vierten Hauptgruppe oder der ersten bis achten Nebengruppe des Periodensystems bedeuten.

2. Verfahren nach Anspruch 1, dadurch gekennzeichnet, daß man die Reaktion bei einer Temperatur von +50 bis +200°C durchführt.

3. Verfahren nach Anspruch 1, dadurch gekennzeichnet, daß man die Reaktion bei einer Temperatur von +100 bis +150°C durchführt.

4. Verfahren nach Anspruch 1, dadurch gekennzeichnet, daß es sich um ein polares, aprotisches Lösungsmittel handelt.

5. Verfahren nach einem der Ansprüche 1 bis 4, dadurch gekennzeichnet, daß man in Gegenwart eines Phasentransferkatalysators arbeitet.

6. Verfahren nach einem der Ansprüche 1, 4 oder 5, dadurch gekennzeichnet, daß man in Gegenwart eines absolutierten Lösungsmittels arbeitet.

7. Verfahren nach einem der Ansprüche 1 bis 6, dadurch gekennzeichnet, daß die Reaktion unter Schutzgasatmosphäre durchgeführt wird.

8. Verfahren zur Herstellung gemäß Anspruch 1 eines 3-(N-anilino)-tetrahydrothiophen-2-on-Derivats der Formel I

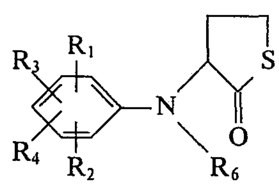

(I)

worin

$R_1$ Wasserstoff, $C_1$—$C_4$-Alkyl, $C_1$—$C_4$-Alkoxy oder Halogen,
$R_2$ Wasserstoff, $C_1$—$C_4$-Alkyl, $C_1$—$C_4$-Alkoxy, Halogen oder Nitro,
$R_3$ Wasserstoff, $C_1$—$C_4$-Alkyl, $C_1$—$C_4$-Alkoxy, Halogen oder die Gruppe $CH_2$—X—$R_8$ bedeuten, wobei $R_8$ für $C_1$—$C_4$-Alkyl, Phenyl oder Benzyl und X für Sauerstoff oder Schwefel stehen,
$R_4$ Wasserstoff oder $C_1$—$C_4$-Alkyl,
$R_6$ Wasserstoff oder die Gruppe —CO—$R_7$ bedeutet, wobei $R_7$ für einen gesättigten oder ungesättigten aliphatischen Kohlenwasserstoffrest mit maximal 4 Kohlenstoffatomen oder eine Alkylthioalkyl- oder Alkoxyalkylgruppe mit 2 bis 5 Kohlenstoffatomen, eine durch Halogen substituierte oder unsubstituierte 2-Furyl- oder 2-Tetrahydrofurylgruppe, eine 1,2,4-Triazolylmethyl- oder Cyclopropylgruppe steht,

gekennzeichnet durch die Reaktion eines 3-(N-anilino)-tetrahydrofuran-2-on-derivats der Formel II

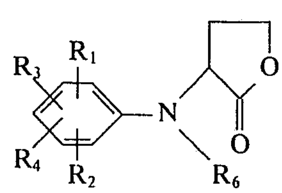

(II)

worin $R_1$, $R_2$, $R_3$, $R_4$ und $R_6$ die unter Formel I angegebenen Bedeutungen haben, in einem organischen

Lösungsmittel mit einem Thiocarbonsäuresalz der Formel III

R—COSX (III)

worin R für eine substituierte oder unsubstituierte Alkyl- oder Arylgruppe steht und X ein Metall- oder ein Ammoniumion bedeutet.

9. Verfahren nach Anspruch 8 gemäß einem der Ansprüche 2 bis 7.

10. Verfahren nach einem der Ansprüche 1 oder 8, dadurch gekennzeichnet, daß X für ein Alkali- oder Erdalkalikation steht.

11. Verfahren nach einem der Ansprüche 1 oder 8, dadurch gekennzeichnet, daß X für $NH_4^{\oplus}$, $NH_3(Alkyl)^{\oplus}$, $NH_2(Alkyl)_2^{\oplus}$, $NH(Alkyl)_3^{\oplus}$ oder $N(Alkyl)_4^{\oplus}$ steht, wobei der Alkylteil 1 bis 12 Kohlenstoffatome enthält.

12. Verfahren nach einem der Ansprüche 1 oder 8, dadurch gekennzeichnet, daß das Salz einer $C_1$—$C_6$-Alkyl-Thiocarbonsäure eingesetzt wird.

## Claims

1. A process for the preparation of a 3-(N-arylamino)-tetrahydrothiophen-2-one derivate of the general formula A

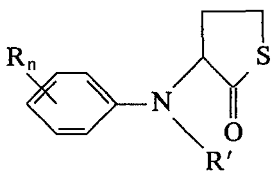

(A)

in which the heterocyclic five-membered ring is unsubstituted or substituted by one or more $C_1$—$C_4$-alkyl groups, n is an integer from 0 to 5, R signifies a number, spezified by n, of substituents selected from the group consisting of $C_1$—$C_{12}$alkyl, $C_1$—$C_{12}$alkoxy, halogen, nitro and $—CH_2—X—R''$, or in which two substituents R which are adjacent to each other, together with the phenyl group, form a naphthyl ring, X being oxygen or sulfur and R″ being $C_1$—$C_{12}$alkyl, phenyl or benzyl, each of which is unsubstituted or substituted by one or more members selected from the group consisting of lower alkyl, lower alkoxy, halogen and nitro, and R′ is hydrogen or the —CO—R‴ group, in which R‴ is an aliphatic, heterocyclic-aliphatic or heterocyclic radical, each heterocyclic structure containing one or more N, O and/or S atoms, or in which R″ is an aromatic radical or an araliphatic radical, each of which radicals may be halogenated in the aromatic moiety and an aliphatic chain in any one of the substituents R‴ may be interrupted by one or more oxygen, sulfur or nitrogen atoms; which process comprises reacting a compound of formula B

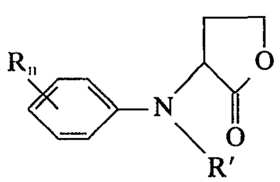

(B)

in which the possible substituents are as defined for formula A, with a salt of a thiocarboxylic acid of formula III

R—COS—X (III)

in which R is a substituted or unsubstituted aliphatic or aromatic radical and X is a cation from the first to fourth main groups or the first to eight subgroups of the Periodic Table, in an organic solvent and at a temperature in the range from about +15 to +200°C.

2. Aprocess according to claim 1, wherein the reaction is carried out at a temperature in the range from +50 to +200°C.

3. A process according to claim 1, wherein the reaction is carried out at a temperature in the range from +100 to +150°C.

4. A process according to claim 1, wherein the solvent is a polar, aprotic solvent.

5. A process according to any one of claims 1 to 4, wherein the reaction is carried out in the presence of a phase transfer catalyst.

6. A process according to any one of claims 1, 4 or 5, wherein the reaction is carried out in the presence of a solvent which has been rendered absolutely dry.

7. A process according to any one of claims 1 to 6, wherein the reaction is carried out in an inert gas atmosphere.

8. A process atmosphere for the preparation, according to claim 1, of a 3-(N-anilino)tetrahydrothiophen-2-one derivative of the formula I

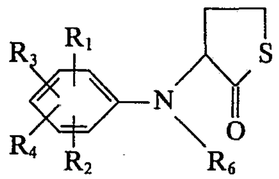

(I)

in which $R_1$ is hydrogen, $C_1$—$C_4$alkyl, $C_1$—$C_4$alkoxy or halogen, $R_2$ is hydrogen, $C_1$—$C_4$alkyl, $C_1$—$C_4$alkoxy, halogen or nitro, $R_3$ is hydrogen, $C_1$—$C_4$alkyl, $C_1$—$C_4$alkoxy, halogen or the group $CH_2$—X—$R_8$, in which $R_8$ is $C_1$—$C_4$alkyl, phenyl or benzyl and X is oxygen or sulfur, $R_4$ is hydrogen or $C_1$—$C_4$alkyl, and $R_6$ is hydrogen or the group —CO—$R_7$, in which $R_7$ is a saturated or unsaturated aliphatic hydrocarbon radical having not more than 4 carbon atoms or is an alkylthioalkyl or alkoxyalkyl group having 2 to 5 carbon atoms, a 2-furyl or 2-tetrahydrofuryl group which is unsubstituted or substituted by halogen, or is a 1,2,4-triazolylmethyl or cyclopropyl group, which process comprises reacting a 3-(N-anilino)tetrahydrofuran-2-one derivative of the formula II

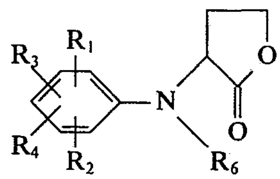

(II)

in which $R_1$, $R_2$, $R_3$, $R_4$ and $R_6$ are as defined for formula I, with a thiocarboxylic acid salt of the formula III

R—COSX (III)

in which R is a substituted or unsubstituted alkyl or aryl group and X is a metal ion or an ammonium ion, in an organic solvent.

9. A process according to claim 8, in accordance with any one of claims 2 to 7.

10. A process according to either of claims 1 or 8, wherein X is an alkali metal cation or alkaline earth metal cation.

11. A process according to either of claims 1 or 8, wherein X is $NH_4^{\oplus}$, $NH_3(alkyl)^{\oplus}$, $NH_2(alkyl)_2^{\oplus}$, $NH(alkyl)_3^{\oplus}$ or $N(alkyl)_4^{\oplus}$, the alkyl moiety containing 1 to 12 carbon atoms.

12. A process according to either of claims 1 or 8, wherein a salt of $C_1$—$C_6$alkylthiocarboxylic acid is used.

## Revendications

1. Procédé de préparation de dérivés de 3-(N-arylamino)-tétrahydrothiophène-2-ones de formule générale A

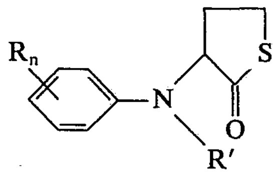

(A)

dans laquelle le noyau hétérocyclique à 5 chaînons est non substitué ou mono-ou poly-substitué par des groupes alkyle en C 1—C 4, n est un nombre entier de 0 à 5, R représente des substituants alkyle en C 1—C 12, alcoxy en C 1—C 12, halogéno, nitro ou —$CH_2$—X—R″ dont le nombre est déterminé par n, ou bien deux substituants R en positions voisines forment, avec le groupe phényle, un cyclecycle naphtyle, X représente l'oxygène ou le soufre, R″ représente un groupe alkyle en C 1—C 12, phényle ou benzyle, chacun de ces groupes pouvant être non substitué ou mono- ou poly-substitué par des groupes alkyle inférieurs, alcoxy inférieurs, des halogènes ou des groupes nitro, R′ représente l'hydrogène ou le groupe —CO—R″ dans lequel R‴ est un reste aliphatique, hétérocyclique-aliphatique ou hétérocyclique, l'hétérocycle pouvant contenir dans chaque cas un ou plusieurs atomes de N, de O et/ou de S, ou bien R‴ représente un reste aromatique ou araliphatique qui peuvent chacun être halogéné dans la partie

aromatique, une chaîne aliphatique présente dans l'un quelconque des substituants R''' pouvant être interrompue une ou plusieurs fois par l'oxygène, le soufre ou l'azote; ce procédé se caractérisant en ce que l'on fait réagir un composé de formule B

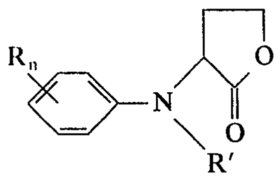

(B)

dans laquelle les substituants éventuels ont les significations indiquées en référence à la formule A, dans un solvant organique, à des températures de +15 à +200°C environ, avec un sel d'un acide thiocarboxylique de formule III

R—COS—X (III)

dans laquelle R représente un reste aliphatique ou aromatique substitué ou non et X représente un cation du premier au quatrième groupe principal ou du premier au huitième sous-groupe de la Classification Périodique.

2. Procédé selon la rev. 1, caractérisé en ce que l'on effectue la réaction à une température de +50 à+200°C.

3. Procédé selon la rev. 1, caractérisé en ce que l'on effectue la réaction à une température de +100 à +150°C.

4. Procédé selon la rev. 1 caractérisé en ce que l'on utilise un solvant polaire aprotonique.

5. Procédé selon les rev. 1 à 4, caractérisé en ce que l'on opère en présence d'un catalyseur à transfert de phase.

6. Procédé selon l'une des rev. 1, 4 ou 5, caractérisé en ce que l'on opère en présence d'un solvant anhydre.

7. Procédé selon l'une des rev. 1 à 6, caractérisé en ce que l'on effectue la réaction en atmosphère de gaz protecteur.

8. Procédé pour la préparation selon la rev. 1 d'un dérivé de la 3-(N-anilino)-tétrahydrothiophène-2-one, de formule I

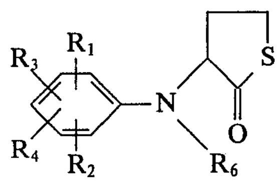

(I)

dans laquelle

$R_1$ représente l'hydrogène, un groupe alkyle en C 1—C 4 ou un halogène,
$R_2$ représente l'hydrogène, un groupe alkyle en C 1—C 4, alcoxy en C 1—C 4, un halogène ou un groupe nitro,
$R_3$ représente l'hydrogène, un groupe alkyle en C 1—C 4, alcoxy en C 1—C 4, un halogène ou le groupe $CH_2$—X—$R_8$ dans lequel $R_8$ représente un groupe alkyle en C 1—C 4, phényle ou benzyle et X représente l'oxygène ou le soufre,
$R_4$ représente l'hydrogène ou un groupe alkyle en C 1—C 4,
$R_6$ représente l'hydrogène ou le groupe —CO—$R_7$ dans lequel $R_7$ représente un reste hydrocarboné aliphatique saturé ou non saturé contenant au maximum 4 atomes de carbone ou un groupe alkylthioalkyle ou alcoxyalkyle en C 1—C 5, un groupe 2-furyle ou 2-tétrahydrofuryle substitué par des halogènes ou non substitués, un groupe 1,2,4-triazolylméthyle ou cyclopropyle,

caractérisé en ce que l'on fait réagir un dérivé de la 3-(N-anilino)-tétrahydrofuranne-2-one, de formule II

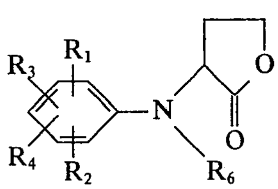

(II)

dans laquelle $R_1$, $R_2$, $R_3$, $R_4$ et $R_6$ ont les significations indiquées en référence à la formule I, dans un solvant organique, avec un sel d'acide thiocarboxylique de formule III

$$R—COSX \quad (III)$$

dans laquelle R représente un groupe alkyle ou aryle substitué ou non et X un ion métallique ou un ion ammonium.

9. Procédé selon la rev. 8, selon l'une des rev. 2 à 7.

10. Procédé selon l'une des rev. 1 ou 8, caractérisé en ce que X représente un cation alcalin ou alcalino-terreux.

11. Procédé selon l'une des rev. 1 ou 8, caractérisé en ce que X représente $NH_4^+$, $NH_3(alkyle)^+$, $NH_2(alkyle)_2^+$, $NH(alkyle)_3^+$ ou $N(alkyle)_4^+$, la partie alkyle contenant 1 à 12 atomes de carbone.

12. Procédé selon l'une des rev. 1 ou 8, caractérisé en ce que l'on met en oeuvre le sel d'un acide (alkyle en C 1—C 6)-thiocarboxylique.